Europäisches Patentamt

(19) European Patent Office

Office européen des brevets

(11) EP 0 408 637 B1

## (12) EUROPEAN PATENT SPECIFICATION

(45) Date of publication and mention
of the grant of the patent:
**09.10.1996 Bulletin 1996/41**

(21) Application number: **89904489.5**

(22) Date of filing: **13.03.1989**

(51) Int. Cl.$^6$: **A61B 5/00**, A61B 5/103

(86) International application number:
**PCT/US89/00997**

(87) International publication number:
**WO 89/08428 (21.09.1989 Gazette 1989/23)**

(54) **NEAR-INFRARED ANALYSIS OF TISSUE FAT PERCENTAGE**

MESSUNG DES FETTANTEILS IM GEWEBE MITTELS NAHE DEM INFRAROTBEREICH
GELEGENER LICHTSTRAHLUNG

ANALYSE UTILISANT UN RAYONNEMENT PROCHE DES INFRAROUGES POUR DETERMINER
LE POURCENTAGE DE GRAISSE DANS UN CORPS

(84) Designated Contracting States:
**AT BE CH DE FR GB IT LI LU NL SE**

(30) Priority: **14.03.1988 US 167711**
**03.10.1988 US 252548**

(43) Date of publication of application:
**23.01.1991 Bulletin 1991/04**

(73) Proprietor: **FUTREX, INC.**
**Gaithersburg, MD 20886 (US)**

(72) Inventor: **ROSENTHAL, Robert, D.**
**Gaithersburg, MD 20879 (US)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**US-A- 3 769 974**      **US-A- 4 633 087**
**US-A- 4 796 633**      **US-A- 4 801 804**

**Description**

BACKGROUND OF THE INVENTION

Field of the Invention

The present invention relates to improvements in instruments and methods for performing near infrared quantitative analysis to determine percent fat in a body.

Description of the Background Art

It has long been known that obesity reduces longevity, and recent studies have demonstrated that high percentage of body fat is an independent health risk factor as a cause of heart attack, stroke, diabetes and other disabling diseases. (Stokes et al, Metabolic Complications of Human Obesities; Elsevier Science Publishers, B.V. (Biomedical Division); J. Vague et al, eds.; pp. 49-57 [1985]).

For the above reasons, several techniques have been developed to determine percent body fat, including recent techniques based on USDA research that demonstrates that "near-infrared light interactance" can provide the basis for measurement of percent body fat (Conway et al, The American Journal of Clinical Nutrition 40:1123-1130 [1984]).

Near-infrared light interactance technology disclosed in U. S. Patent No. 4,633,087 to Rosenthal et al has recently been utilized in a commercial instrument for measurement of body composition, i.e., percent fat in the human body. However, because of the cost required to manufacture an instrument that utilizes this technology, the majority of purchasers are health clubs, medical centers and sports teams, with only a very small percentage of buyers being individual consumers.

Taking full advantage of the technology disclosed in U.S. Patent No. 4,633,087 requires the measurement of more than one wavelength in the near-infrared spectrum. The reason for this is that what is being measured is the change in slope of the absorption curve, with the slope being defined as the difference in optical absorption at two defined wavelengths.

For the following reasons, the cost of utilizing the technology described in U.S. Patent No. 4,633,087 remains high even when utilizing inexpensive infrared emitting diodes (IREDs) as the near-infrared source:

(1) The use of two IREDs are preferred for each of two wavelengths being measured, and the more IREDs that are used, the greater the expense.
(2) An electronic means for turning on and off each pair of IREDs in a sequential fashion and keeping them on for a predetermined length of time is required.
(3) Circuitry is required that allows the output of the pairs of IREDs to be adjusted so that they have equal energies when measuring a neutral sample.
(4) Computation circuitry is required that must not only discriminate between two pairs of IREDs, but also perform a multiple regression calculation.
(5) Instrument display capability is required that has the ability to read-out each of the two pairs of IREDs, as well as the final percent fat.
(6) The instrument must also have the ability of entering a multiple number of constants because of the multi-term linear regression equation utilized.

In addition to each of the items discussed above, a major element in the production cost of current near-infrared analysis instruments is the need to calibrate each production unit against a series of known samples via multiple linear regression analyses. These calibration steps are labor intensive and their elimination would enable great reductions in the cost of producing such instruments.

In view of the costs required in providing known devices for measuring body fat content, there remains a need in the art for improved and less expensive devices for measuring percent body fat.

SUMMARY OF THE INVENTION

In accordance with the present invention, a method for determining percent fat in a body comprises transmitting substantially uniformly dispersed near-infrared radiation into a body to achieve optical interactance between the body and the near-infrared radiation. Optical absorption by the body of the near-infrared radiation is measured at only one wavelength of the near-infrared radiation. The measured absorption at that one wavelength of the near-infrared radiation is utilized to quantitatively determine the fat content of the body. Data on a plurality of physical parameters of the body, such as height, weight, exercise level, sex, and race, may be utilized along with the measured absorption, to quantitatively determine the fat content of the body. The invention further relates to an apparatus for carrying out the above-described method.

In accordance with another aspect of the present invention, a method for determining percent fat in a body comprises placing a point source of near-infrared radiation against a body, transmitting near-infrared radiation into the body, detecting near-infrared radiation which interacted with the body and providing a readout, based on near-infrared absorption by the body during interactance, indicative of body fat content. Placing the point source against the body eliminates the need for the light-diffusing probes.

The invention further relates to apparatus for quantitatively measuring fat content of a body comprising at least one point source means of near-infrared radiation, a near-infrared detector capable of providing an electrical signal upon detection of near-infrared radiation, and means for placing the point source means against the body so as to introduce near-infrared radiation for absorption measurement. Data on a plurality of physical parameters, especially height and weight, again may be utilized along with the measured absorption to quantitatively determine fat content.

## BRIEF DESCRIPTION OF THE DRAWINGS

Fig. 1 is a partially schematic perspective view of an instrument according to the prior art.

Fig. 2 is a detailed sectional, partially schematic view of a portion of the instrument of Fig. 1 showing IRED positioning.

Fig. 3 is a detailed sectional, partially schematic, elevation view of the lower end of the instrument shown in Fig. 1.

Fig. 4A graphically shows optical density at 937 nanometers of the biceps of fourteen human subjects, versus percent body fat.

Fig. 4B graphically shows optical density at 947 nanometers of the biceps of fourteen human subjects, versus percent body fat.

Fig. 5 is a sectional, partially schematic view of an instrument according to the present invention.

Fig. 6 is a sectional, partially schematic view of the instrument of Fig. 5 in combination with a calibration sleeve.

Fig. 7 is a plot of linear voltage output from an optical detector versus percent body fat of a subject.

Fig. 8 is a plot of linear voltage output from an optical detector versus energy received from an IRED point source.

## DETAILED DESCRIPTION OF THE PREFERRED EMBODIMENTS

The invention provides method and apparatus for determining percent body fat utilizing optical interactance principles in the near-infrared radiation wavelength range of from about 740 to about 1100 nanometers. Because of the previously unknown relationship between optical density (O.D.) and percent body fat, O.D. measurement of a single bandwidth of near-infrared radiation can be utilized to provide a high correlation with percent body fat.

Optical density ordinarily is defined as log 1/I, wherein I is interactance and equal to $E_s/E_r$ ($E_s$ = energy received from subject; $E_r$ = energy received from a reference). An important aspect of the present invention is the optional substitution of much simpler 1/I mathematics for the conventional log (1/I) mathematics. When taking a measurement halfway between the shoulder and elbow on the biceps of a person's prominent arm (the one used for writing), the local amount of fat measured is directly proportional to the total fat in the body. With the present invention, a single bandwidth measurement can provide meaningful measurement of percent total body fat. A single bandwidth is able to provide this measurement since the higher the percent body fat, the more transparent the arm of the subject. This is because low body fat people have "hard muscles" that make it difficult for light to penetrate, therefore providing high O.D. values. Conversely, people with high percent body fat have a "flabby" biceps that is not very optically dense, resulting in low O.D. values.

Although there is no need to be particularly specific in the bandwidth of interest that the IRED emits, so long as it is within the near-infrared spectrum, the larger the half-power bandwidth of the light source, within reason, the better the measurement, since less interference from other body parameters occurs. Thus, the use of a conventional 950 nanometer IRED as the illumination source is almost ideal. Such infrared-emitting diodes have half-power bandwidths of almost 60 nanometers, which make them practically immune to other types of absorptions (e.g., absorption due to moisture, protein, etc.).

This invention utilizes the principal of interactance, which principle is known in the art and differs from reflectance and transmittance. In interactance, light from a source is shielded by an opaque member from a detector and interactance of the light with the test subject is then detected by the detector.

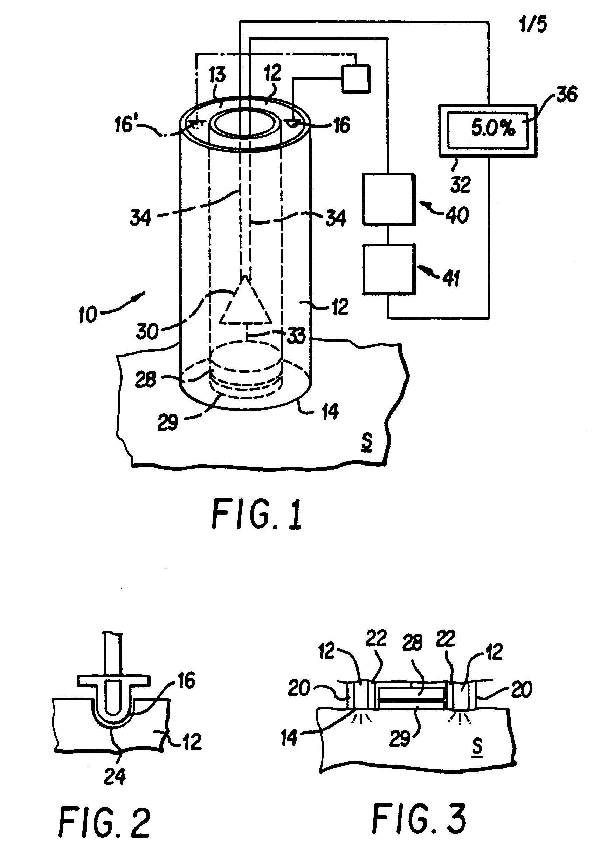

Although there are some similarities between an apparatus in accordance with Figures 1 to 3 and that disclosed in U.S. Patent No. 4,633,08, there are significant differences between this prior art and an embodiment on basis of Fig. 1 to 3. As shown in Fig. 1, the probe portion 10 of the instrument of the invention is of hollow cylindrical form and includes a hollow tubular member 12 having a wall of solid translucent material selected so that it transmits and does not substantially or inconsistently absorb near-infrared energy in the bandwidth of interest, namely, from about 740 to about 1100 nanometers. Examples of suitable materials out of which tubular member 12 may be constructed include, but are not limited to, translucent nylon, translucent polytetrafluoroethylene and the like. Means for providing a point source of near-infrared radiation of a predetermined wavelength is positioned at an upper end portion 13 of tubular member 12. The near-infrared point source means at the upper end portion 13 of tube 12 is positioned so that near-infrared radiation of the predetermined wavelength emitted from the point source means will be transmitted by the tubular member 12 from the upper end portion 13 to a flat bottom surface 14 of tube 12. The near-infrared point source means preferably comprises infrared emitting diode (IRED) means 16.

Although there is no need to be particularly specific in the wavelength of interest that the IRED emits, so long as it is within the near-infrared spectrum, the larger the half-power bandwidth of the light source, within reason, the better the measurement, since less interference from other body parameters occurs. Thus, the use of a conventional 950 nanometer IRED as the illumination source is almost ideal. Such infrared-emitting diodes have half-power bandwidths of almost 60 nanometers, which make then practically immune to other types of absorptions (e.g., absorption due to moisture, protein, etc.).

In preferred embodiments, light transmitting tube 12 is made a suitable length to provide sufficient internal light scattering to smooth out the emitted light so that light from the IRED is transmitted through tube 12 and emerges uniformly at the bottom surface 14 of the tube. Most preferably, the tube 12 is no longer than is necessary to uniformly smooth out the light emitted from the IRED, in order to minimize the loss of near-infrared radiation. The ideal tube length can be easily determined by utilizing a commercially available infrared viewer (nightscope). A tube may be sized by observing near-infrared radiation passing through the tube and trimming the tube until the light emerges uniformly. A silicon detector is then passed around the end of the tube to check for uniform output.

The tube can be shorter than is required for uniform light emergence. If the tube length is such that non-uniform light emerges from the end of the tube, consistent results can still be obtained if the tube is oriented in the same direction for each reading, so that the non-uniformity of emerging light will be consistently read.

Since only one IRED is required, to achieve desirably uniform light emergence, tube 12 must be longer than that described in U.S. Patent No. 4,633,087, e.g., about 1.5 times longer than when using a pair of IREDs. Longer tube length is acceptable since a single wavelength measurement does not require the precision that is required in the preferred embodiment of the above-described patent. When a two wavelength measurement is made, as in the preferred embodiment of U.S. Patent No. 4,633,087, a small change in the energy between the two wavelengths must be resolved. Thus, to make an accurate measurement of body fat, a two wavelength measurement requires the ability to resolve approximately 0.001 difference between optical density (log 1/I values) at two wavelengths. Thus, the precision of the measurement must be relatively high, requiring a twelve bit analog-to-digital converter.

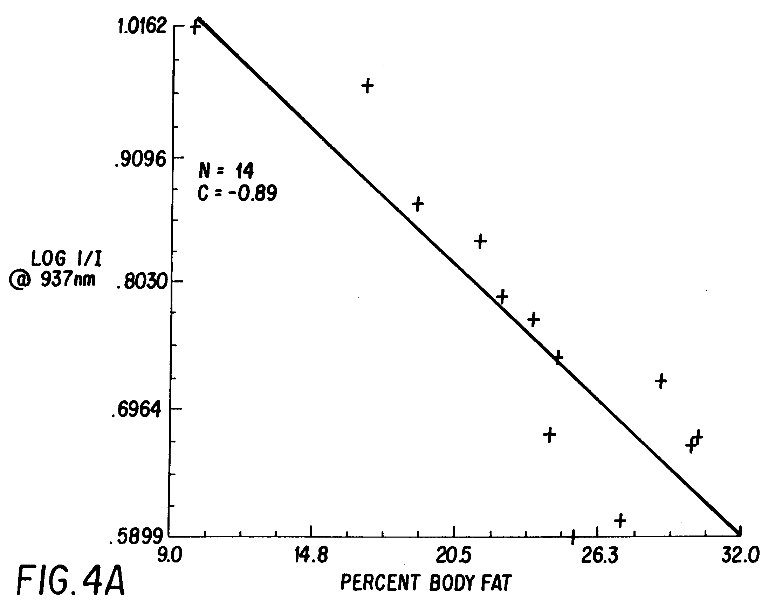

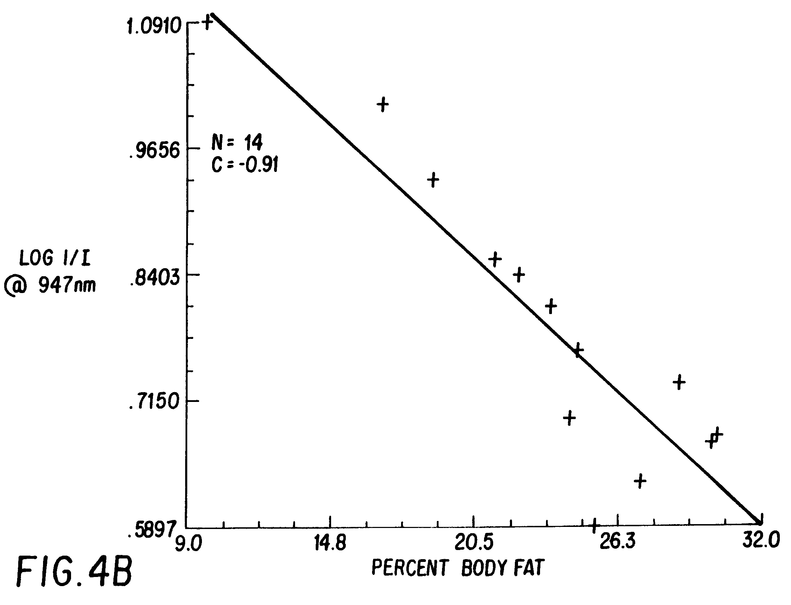

Figs. 4A and 4B represent single wavelength measurements for fourteen subjects at 937 nanometers and 947 nanometers, respectively, and show the negative correlation of percent body fat and optical density. In a single wavelength measurement, the resolution can be at least ten times less stringent, and perhaps as much as one hundred times less stringent, than is required using a two wavelength measurement (i.e., an eight-bit analog-to-digital conversion is acceptable). Thus, the lower resolution means allows a lower light level, which in turn permits the use of a single IRED in conjunction with a longer light tube 12. If desired, however, more than one IRED emitting the same wavelength can be used to increase the light level, such as IREDs 16, 16', but it is only necessary to measure absorbance at a single wavelength emitted by both diodes.

For light shielding purposes, the cylindrical walls of tubular light transmitting member 12 are shielded on the outside by an outer tubular opaque shield 20 and on the inside by inner tubular opaque shield 22. The upper end portion 13 of tubular member 12 is also shielded from ambient light by a top cover, not shown.

In the embodiment shown, IRED 16 is positioned in a depression 24 in the top surface of the upper end portion 13 of light-transmitting tube 12. See Fig. 2.

An optical detector 28, capable of detecting near-infrared radiation, is positioned inside of and at the bottom end portion of the tubular member 12 as shown in Figs. 1 and 3. Inner tubular shield 22 is positioned between detector 28 and transmitting tube 12, thereby providing an opaque mask which prevents near-infrared radiation from tube 12 from impinging directly on detector 28. Optical detector 28 generates an electrical signal when the detector detects light.

The optical detector 28 is connected to the input of an electrical signal amplifier 30 by suitable electrical conducting means 33. Amplifier 30 may be an inexpensive signal amplifier, and amplifies signals generated by detector 28 in response to light detected by the detector. The detector 28 preferably is positioned within tube 22. The output of amplifier 30 feeds the amplified signal generated by detector 28 to a readout box 32 through conductive lines 34. The readout box 32 may have a display 36 for directly reading the percentage of fat in a test subject S.

A near-infrared-transparent window 29 is located in front of the optical detector 28. If desired, window 29 can be electrically conductive and grounded directly to the apparatus electronics to provide shielding from electro-magnetic interferences that are commonly encountered in industrial and consumer premises. However, acceptable results are achievable when using non-electrically conductive windows.

The output of amplifier 33 is fed to an integrating analog-to-digital converter 40 having an eight bit output, which is connected to a digital processor 41 connected to readout box 36.

The data processing and readout means connected to amplifier 30 are capable of processing the amplified signal resulting from detection of only a single, predetermined wavelength, to provide a readout indicative of the percent fat in the body based on the detection of that single, predetermined wavelength.

Since the present invention measures radiation of only a single near-IR bandwidth (which may be emitted from only a single IRED), there is no need for the instrument to cycle on and off, as is required when utilizing multiple bandwidth measurements. Thus, there is no need for the inclusion of a timing circuitry nor IRED cycling circuitry, as is provided in a multiple bandwidth instrument.

In operation, the bottom surface 14 and window 29 are positioned against a surface of test subject S. Substantially uniformly dispersed near-infrared radiation emerging from end 14 is transmitted into the body of test subject S to achieve optical interactance between the body and the near-infrared radiation. Near-infrared radiation is detected by detector 28, and optical absorption by the body at only one predetermined wavelength of the near-infrared radiation is measured. Detector 28 then generates an electrical signal representing the measured absorption at only the one predetermined wavelength, which is thereafter utilized to quantitatively determine the fat content of the body.

With only a single wavelength measurement, a simple slope/bias computation is all that is required to directly determine percent fat. Accordingly, the computation required with single wavelength measurement is considerably simpler, and less costly to implement, than the computation required with multiple wavelength measurement. With single wavelength measurement, the equation can be as simple as:

$$\% \text{ body fat} = K_0 + K_1 (1/I)$$

wherein I is as defined above, $K_0$ represents an intercept error constant and $K_1$ represents a line slope constant, both constants being determined by multiple regression techniques, i.e., optical readings are obtained from the components of the instrument being constructed for a representative number of samples which have been previously accurately analyzed, and the optical readings and previously measured percentages are utilized to calculate sets of constant values for fat content using a conventional regression algorithm in a digital computer. The respective K values are then programmed into the analyzing instrument being constructed so that the instrument can directly compute percentage fat from optical data readings.

Performing the analysis on a linear basis in accordance with the equation immediately above substantially reduces the cost of the instrument, but also results in a considerable decrease in accuracy. The accuracy of the measurement can be greatly increased by performing the analysis according to the following equation:

$$\% \text{ body fat} = K0 + K_1 (\log 1/I)$$

wherein $K_0$, $K_1$ and I are as defined above.

As noted above, the single measurement can be made using an IRED at almost any near-infrared center wavelength. However, people of African origin have flesh pigments that absorb light from the visible portion of the spectrum through the very near-infrared spectrum, disappearing at about 950 nanometers. Thus, the commercially available low-cost IREDs at 950 nanometers are practically ideal, since they avoid a substantial effect in the measurement based on skin color.

To provide even more accurate determination of percent body fat, data on a plurality of physical parameters of the body can be utilized along with the measured absorption of near-infrared radiation, to quantitatively determine the fat content of a body. Such physical parameters include, but are not limited to height, weight, exercise level, sex, race, waist-to-hip measurement, and arm circumference. When utilizing data on physical parameters in conjunction with measurement of near-infrared absorption in a single wavelength measurement, a suitable equation is as follows:

$$\% \text{ body fat} = K_0 + K_1 (\log 1/I) + K_3 (W/100) + K_4 (H/100) + K_5 (S) + K_6 (EL)$$

wherein $K_0$, $K_1$ and I are as defined above. W is weight in kg x 0,454 (pounds); H is height in mm x 25.4 (inches); S is sex (males = +0.01, female = -0.01); EL is exercise level: none equals 0; light = 0.02; moderate = 0.05; heavy = 0.08. $K_3 - K_6$ are constants which are determined by multiple regression techniques as described above, i.e., optical readings are obtained from the components of the instrument being constructed for a representative number of samples that have been accurately analyzed, and the optical readings and previously measured percentage are utilized to calculate sets of $K_3 - K_6$ values for the respective body parameters using a conventional regression algorithm in a digital computer. These sets of $K_3 - K_6$ values are then programmed into the analyzing instrument being constructed so that the instrument can directly computer the percentage body fat (taking into consideration both the optical data readings and the data on the physical parameters of the body of a particular subject.)

Data on a plurality of physical parameters of the body can also be utilized in conjunction with multiple wavelength measurement of near-infrared absorbance, as in prior U.S. Patent No. 4,633,087, in accordance with the following formula:

$$\% \text{ body fat} = K_0 + K_{2A} (\log 1/I_1) + K_{2B} (\log 1/I_2) + K_3 (W/100) + K_4 (H/100) + K_5 (S) + K_6 (EL)$$

wherein W, H, S and EL are as defined above; $K_0$ and $K_3$ - $K_6$ are as defined above; and $k_{2A}$ and $K_{2B}$ are the respective slopes of curves representing two wavelengths being measured, and are determined by multiple regression techniques as described above. $I_1$ is interactance at one of the two wavelengths being measured, and $I_2$ is interactance at the other of the two wavelengths being measured. According to this embodiment, one of said wavelengths preferably is about 937 nanometers plus or minus about 2 nanometers, and the other of said wavelengths preferably is about 947 nanometers plus or minus about 2 nanometers, with a minimum of about 10 nanometers between said two wavelengths.

Actual K values for two instruments constructed utilizing multiple wavelength technology in accordance with the formula immediately above are set forth in Table I below:

TABLE I

|  | INSTRUMENT A | INSTRUMENT B |
|---|---|---|
| $K_0$ | 94.3 | 84.2 |
| $K_{2A}$ | -15.5 | -16.3 |
| $K_{2B}$ | -8.0 | -6.2 |
| $K_3$ | 8.3 | 8.1 |
| $K_4$ | -79.0 | -13.7 |
| $K_5$ | -93.9 | -124.2 |
| $K_6$ | -78.7 | -81.4 |
| Correlation | .989 | .989 |
| Std. deviation | .951 | .987 |
| Figure of Merit | 13.5 | 13.0 |

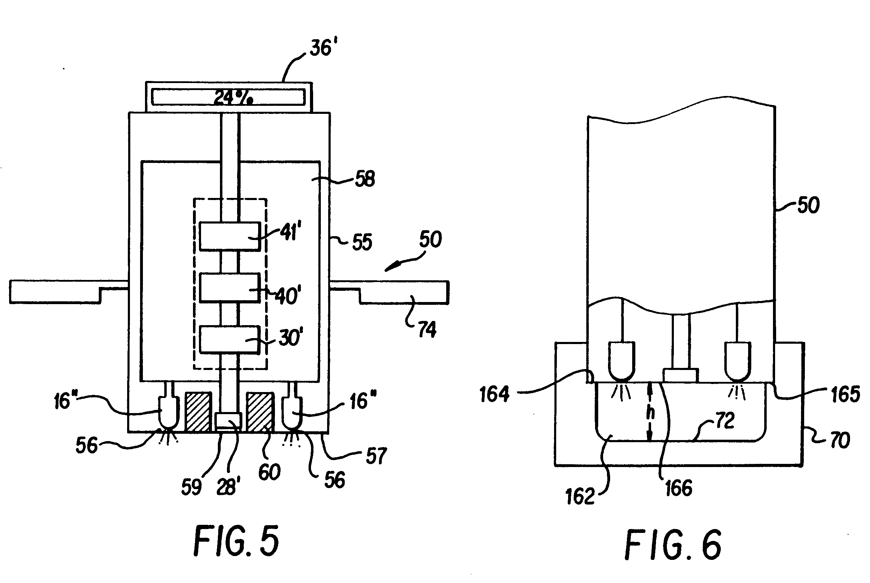

An instrument in accordance with the invention is illustrated in Figure 5. In this embodiment, a light transmitting and diffusing member as taught in connection with many prior devices is not needed. Instead, at least one and preferably a pair of IREDs and an optical detector are positioned within the instrument for placement directly adjacent to the skin of the subject, with substantially no loss of fat measuring accuracy.

The instrument 50 is dimensioned for hand-held operation and includes a case 55 housing one or more IREDs 16", a pair of which are shown in opposite sides of the lower portion thereof. When more than one IRED is employed, they should be of about the same bandwidth and center frequency output. The IREDs are disposed opposite window openings 56 in a bottom surface 57 of the case 55. The windows 56 may further include near-infrared-transparent coverings (not shown) to prevent the entry of dust and dirt into the instrument.

An optical detector 28', also positioned in the lower portion of the case 55, is substantially equidistant from each IRED 16". The detector 28" is disposed within a window opening 59 which, like the windows for the IREDs, may include a covering transparent to near-IR radiation. If desired, the window covering over detector 28" can be electronically conductive to provide EMI shielding. Light baffles 60 are placed between each IRED 16" and the detector 28 to prevent erroneous readings caused by direct impingement of near-IR radiation onto the detector. The baffles 60 are constructed of any opaque and preferably lightweight material. Erroneous readings also are prevented by the provision of a flexible light shield 74 which blocks ambient light from impinging upon the detector.

The detector 28' and each of the IREDs 16" are mounted within the case 55 on a printed circuit (PC) board 58 which also serves as a carrier for the remainder of the electronic components.

The optical detector 28' is connected to the input of an electrical signal amplifier 30' which in turns feeds the amplified signal to an analog-to-digital (A/D) converter 40'. The A/D converter is connected to a digital processor 41' which is connected to a readout box 36' (e.g., liquid crystal display). In a preferred embodiment the A/D converter, microprocessor and liquid crystal display driver circuitry are combined within a single chip (illustrated with dashed lines in Fig. 5) such as the μPD75328 chip available from NEC Electronics, Inc. The use of this single chip, which employs a 4-bit microprocessor with 8-bit A/D circuitry, with no loss of accuracy, greatly reduces the cost of production units. The linear voltage output (V) from detector 28' is data processed into a signal indicative of percent body fat which is then displayed on the readout box.

Figure 6 illustrates use of an optical standard sleeve for "zero adjust" of the instrument by the user just before making readings. The standard is a near-infrared-opaque body or sleeve 70 having a cavity 162 and an internal flange 164 for cooperating with the end 165 of instrument 50. The dimensions are chosen such that the tip 166 of instrument 50 will be a predetermined distance (h) from the bottom portion of cavity 162. This distance is chosen to provide a reflectance value that corresponds to an interactance calibration value ($\%_{REF}$) for which the instrument is being calibrated (which is the function of the material's reflection properties and the geometry of the cavity). The standard reflects sufficient near-infrared radiation emitted from the near-infrared source in the probe to the near-infrared detectors present therein for "zeroing" or standardizing the probe for use in an interactance (measurement) mode. The sleeve 70 includes a reflective surface 72 (which reflects a known amount of near-IR radiation and, preferably reflects an amount of near-IR radiation which is substantially equal to the amount of near-IR radiation transmitted during near-IR interactance from a body of approximately 24% body fat content) at the standard distance (h) from the IREDs and detectors.

At the factory, a single "master unit" is calibrated using linear regression techniques in the conventional manner against a number of samples of known fat content (i.e., samples previously analyzed via another universally accepted technique such as underwater weighing). This calibration procedure provides values for the slope (hereinafter "$C_1$") and y-intercept (hereinafter "$C_0$") of the linear fat-determination equation which is used in the master and programmed into each production unit. In producing production units which are calibrated based upon the calibration of a single master unit, the following assumptions are made:

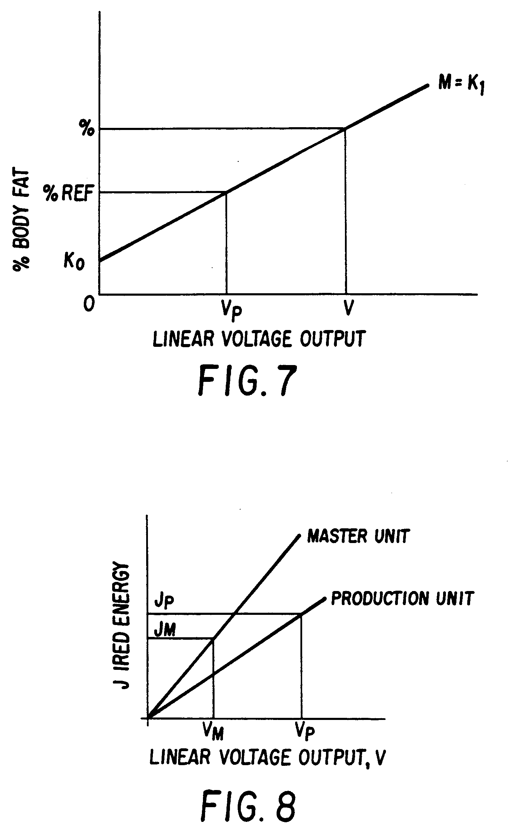

1) The response of all of the linear detectors is linear with respect to light level, and zero voltage is output when light level is zero (see Fig. 8). Each detector has a different sensitivity (i.e. line slope), however, and this sensitivity can change as the detector ages. Thus, a zero adjust step, to calculate the detector line slope and store the value for use during interactance measurement, is to be performed by the user just prior to taking a measurement.
2) The only difference from instrument to instrument is the difference in detector voltage output. This difference can be caused by differences in IRED energy, detector sensitivity, or power supply changes. There is no difference between units due to spectrum characteristics (because the IRED bandwidth is wide) or due to dimension changes.
3) All optical standard sleeves provide identical reflective surfaces so that all sleeves will read the same value (within a few tenths of a percent) on a single instrument.

Following calibration against the known samples, the master unit is fitted with the optical standard sleeve and placed in the zero adjust mode. The readout will display the percent fat value associated with the optical standard according to the formula:

$$\%_{REF} = C_0 + C_1 * V_M \tag{I}$$

where $V_M$ is the linear voltage output from the detector and $C_0$ and $C_1$ are intercept and detector line slope output values, respectively, known from the calibration of the master unit against the known samples. In order for a production unit to provide the same $\%_{REF}$ when the optical standard is measured in zero adjust mode, the following equation must be true:

$$\%_{REF} = K_0 + K_1 * V_P \tag{II}$$

where $K_0$ is an intercept value and $K_1$ is a detector line slope value as seen in Fig. 3. From Fig. 8 and equations I and II the following must be true:

$$\text{when } J_M = 0, \% = C_0 \text{ (master unit)} \tag{III}$$

$$\text{when } J_P = 0, \% = K_0 \text{ (production unit)} \tag{IV}$$

where J is voltage output from the respective detector. As it is desired to have the production units have the same calibration as the master unit, they also must read the same when their detectors output zero voltage. Thus, from (III) and (IV) above:

$$C_0 = K_0 \tag{V}$$

Thus, equation (II) becomes:

$$\%_{REF} = C_0 + K_1 * V_P \tag{VI}$$

Final zeroing of the production unit is to ensure that the unit behaves as closely as possible to the master unit and is carried out by the user in the following manner: The instrument 50 is put in "zero adjust mode" and is positioned within the standard sleeve 70 such that the tip 166 of the instrument 50 is spaced away from the bottom portion of cavity 162 so as to reflect sufficient near-infrared radiation emitted from the tip 166 back to the detector for calibrating the instrument for use in an interactance mode. When "zero adjust" is pressed on the production unit, the unit calculates $K_1$ from (VI) above and stores the $K_1$ value to use when measuring a person.

With only a single bandwidth measurement, a simple slope/bias computation is all that is required to directly determine percent fat. Thus when measuring a person, the equation is:

$$\% = C_0 + (\%_{REF} - C_0) + V_P * V_{SUBJ}$$

where $C_0$, $V_P$ and $\%_{REF}$ are known from above, $V_{SUBJ}$ is the linear output from the detector when measuring the subject person and % is the person's body fat composition.

The material of the optical standard is chosen so that the reflectance characteristics makes it a usable standard for the constituent being measured, such as using polyvinyl chloride (PVC) for the calibration cup as a standard for fat and other types of measurements.

In operation, following calibration, the lower surface of the instrument is placed against the body for interactance measurement. Measurements of greatest accuracy are obtained when the instrument is placed against the biceps and oriented so that the line bisecting the IREDs runs perpendicular to the axis of the arm.

Elimination of the (log 1/I) calculation in favor of the disclosed 1/I based calculation has been shown to result in substantially no loss of accuracy in these interactance measurements. This is because the percent body fat function itself is essentially linear within the measured ranges. Calculations based on this linear function can advantageously be performed with lower cost data processing circuitry than that employed with logarithmic function calculations.

The elimination of costly factory calibration of each production unit in favor of user calibration via the simple zero adjust procedure taught herein also contributes to the lower cost of this preferred embodiment.

As noted above, the single measurement can be made using an IRED at almost any near-infrared center wavelength. However, people of African origin have flesh pigments that absorb light from the visible portion of the spectrum through the very near-infrared spectrum, disappearing at about 950 nanometers. Thus, the commercially available low-cost IREDs which provide a bandwidth output centering on 950 nanometers are practically ideal, since they avoid a substantial effect in the measurement based on skin color.

To provide even more accurate determination of percent body fat, data on a plurality of physical parameters of the body can be utilized along with the measured absorption of near-infrared radiation, to quantitatively determine the fat content of a body. Such physical parameters include, but are not limited to height, weight, exercise level, sex, race, waste-to-hip measurement, and arm circumference. When utilizing data on height and weight parameters in conjunction with measurement of near-infrared absorption in a single bandwidth measurement, a suitable equation is as follows:

$$\% \text{ body fat} = K_0 + (\%_{REF} - C_0) / V_P * V_{SUBJ} + K2 * W/100 * (1 - V_{SUBJ}/V_P) + K_3 * H/100 * (1 - V_{SUBJ}/V_P)$$

where values $K_2$ and $K_3$ are determined for the master unit by multiple linear regression analyses of known subjects as before, W is the subject's weight in pounds and height is his or her height in inches. Other parameters are similarly factored into the above equation.

The present invention provides a method and means for accurately and reliably measuring percent body fat, that is substantially less expensive than with previously known technology, and in a non-destructive manner, using near-infrared radiation interactance principles.

Since many modifications, variations and changes in detail may be made to the described embodiments, it is intended that all matter in the foregoing description and shown in the accompanying drawings be interpreted as illustrative and not in a limiting sense.

## Claims

1. A method for determining percent fat in a body, comprising:

(a) providing a near-infrared quantitative instrument (50) comprising:

(i) at least one point source of near-infrared radiation (16");

(ii) a near-infrared radiation detector (28') capable of providing an electrical signal upon detection of near-infrared radiation;

(iii) means (60) for preventing near-infrared radiation from said at least one point source (16") from impinging directly on said detector (28'); and

(iv) means for converting (30',40',41',36') an electrical signal corresponding to the fat content of the body into readout indicative of the percent fat of the body;

(b) placing the at least one point source (16") against a body to be tested with the detector (28') positioned for receiving near-infrared radiation that interacts with said body;

(c) transmitting near-infrared radiation into said body;

(d) detecting near-infrared radiation which interacted with said body;

(e) providing a readout of percent body fat corresponding to an electrical signal indicative of near-infrared absorption provided by said detector (28')

**characterized by**
providing a near-infrared quantitative instrument (50) further comprising locating means for placing said at least one point source (16") directly against a body to be tested so as to introduce near-infrared radiation into said body, wherein said locating means positions said detector (28') for receiving near-infrared radiation from said at least one point source (16") that interacts with said body and for providing the electrical signal corresponding to the fat content of said body.

2. A method of claim 1, wherein said near-infrared radiation is within the range of about 740 - 1100 nanometers.

3. A method of claim 1, wherein near-infrared absorption is measured at about 950 nanometers.

4. A method of claim 1, wherein near-infrared absorption is measured at two different wavelengths.

5. A method of claim 4, wherein one of said wavelengths is about 937 nanometers plus or minus about 2 nanometers, and the other of said wavelengths is about 947 nanometers plus or minus about 2 nanometers, with a minimum of about 10 nanometers between said two wavelengths.

6. A method of claim 1, wherein data on a plurality of physical parameters of the body are utilized along with said absorption to quantitatively determine the fat content of the body.

7. A method of claim 6, wherein said physical parameters are selected from the group consisting of height, weight, exercise level, sex, race, waist-to-hip measurement, arm circumference, and combinations thereof.

8. A method of claim 7, wherein said physical parameters are height and weight.

9. A near-infrared quantitative instrument (50) for measuring fat content of a body to be tested, comprising:

(a) at least one point source (16") of near-infrared radiation;

(b) a near-infrared radiation detector (28') capable of providing an electrical signal upon detection of near-infrared radiation;

(c) means (60) for preventing near-infrared radiation from said at least one point source from impinging directly on said detector (28'); and

(d) means for converting (30',40',41',36) an electrical signal corresponding to the fat content of the body into a readout indicative of the percent fat of the body

**characterized in that**
the instrument (50) further comprises locating means for placing said at least one point source (16") directly against a body to be tested so as to introduce near-infrared radiation into said body, wherein said locating means positions said detector (28') for receiving near-infrared radiation from said at least one point source (16") that interacts with said body and for providing the electrical signal corresponding to the fat content of said body.

9

10. An instrument of claim 9, wherein said at least one point source of near-infrared radiation comprises an IRED (16").

11. An instrument of claim 9, wherein said at least one point source comprises an IRED (16") and a near-infrared-transparent window covering means (56), for placement against a body to be tested, disposed adjacent said IRED (16").

12. An instrument of claim 9, further comprising a case (55), said locating means comprising a portion of said case having said at least one point source (16") and said detector (28') disposed therein.

13. An instrument of claim 9, further comprising:

a case (55);

said at least one point source (16") of near-infrared radiation being disposed in one end of said case (55), said one end of said case (55) comprising said locating means for positioning said at least one point source against a body to be tested;

said near-infrared radiation detector (28') being positioned adjacent said at least one point source (16") in said one end of said case (55); and

means (30') connected to the detector (28') for amplifying said electrical signal provided by said detector (28').

14. An instrument of claim 9, wherein said at least one point source (16") of near-infrared radiation emits radiation within the range of about 740 - 1100 nanometers.

15. An instrument of claim 9, wherein said at least one point source (16") of near-infrared radiation emits radiation at about 950 nanometers.

16. An instrument of claim 9, further comprising a second point source (16") of near-infrared radiation.

17. An instrument of claim 16, wherein one point source (16") emits radiation at about 937 nanometers plus or minus about 2 nanometers, and the other point source (16") emits radiation at about 947 nanometers plus or minus about 2 nanometers, with a minimum of about 10 nanometers between said two wavelengths.

18. An instrument of claim 9, wherein said means for converting (30',40',41',36') utilizes data on a plurality of physical parameters of the body to quantitatively determine the fat content of the body.

19. An instrument of claim 18, wherein said physical parameters are selected from the group consisting of height, weight, exercise level, sex, race, waist-to-hip measurement, arm circumference and combinations thereof.

20. An instrument of claim 18, wherein said physical parameters are height and weight.

21. In combination, a near-infrared quantitative instrument for measuring fat content of a body to be tested and a reflective standard, comprising:

(a) a case (55);

(b) a near-infrared radiation detector capable of providing an electrical signal upon detection of near-infrared radiation;

(c) means for preventing (60) near-infrared energy from each of said point source means (16") from impinging directly on said detector (28');

(d) means (30') connected to the detector (28') for amplifying an electrical signal provided by said detector (28'); and

(e) means for data processing and readout (40'41',36') connected to the amplifying means (30') and being capable of processing the amplifying signal so as to provide a readout indicative of the percent fat in the body based on detection of said near-infrared radiation, wherein said near-infrared radiation is within the range of about 740 - 1100 nanometers a single pair of closely matched, near-infrared radiation-producing point source means (16") disposed in one end of said case (55), for transmitting near-infrared radiation into the body, said one end of said case (55) comprising locating means for positioning said pair of point source means (16") directly against a body to be tested;

the detector (28') being positioned between each of said point source means (16") in said one end of said case (55); and

a reflective standard sleeve means (70) for positioning over said one end, said sleeve means (70) comprising a reflective surface (72) of a known, predetermined reflectance.

22. A combination of claim 21, wherein said reflective surface (72) reflects near-infrared energy in an amount substantially equal to an amount of near-infrared energy transmitted during near-infrared interactance from a body of about 24% body fat content.

23. A method of claim 1, wherein the step of detecting near-infrared radiation comprises detecting near-infrared radiation at only one wavelength.

## Patentansprüche

1. Verfahren zur Messung des prozentualen Körperfettanteils mit den Schritten:

   a) Zurverfügungstellen eines Nahinfrarot-Mengenmeßgerätes (50), enthaltend: (i) wenigstens eine Punktlichtquelle mit einer Strahlung im nahen Infrarot (16"), (ii) ein Nahinfrarot-Strahlungsdetektor (28'), der in der Lage ist, nach Empfang einer Nahinfrarotstrahlung ein elektrisches Signal auszugeben, (iii) eine Einrichtung (60), die verhindert, daß Nahinfrarotstrahlung von der wenigstens einen Punktlichtquelle (16") direkt auf den Detektor (28') auftrifft; und (iv) eine Einrichtung (30',40',41',36') zum Wandeln eines elektrischen Signals, das dem Körperfettanteil entspricht, in einen Ausgabewert, der den prozentualen Körperfettanteil angibt;
   b) Anlegen wenigstens einer Punktlichtquelle (16") an einen zu überprüfenden Körper, wobei der Detektor (28') derart angeordnet ist, daß Nahinfrarotstrahlung, die mit dem Körper in Wechselwirkung steht, empfangen wird;
   c) Übertragen von Nahinfrarotstrahlung in den Körper;
   d) Erfassen der Nahinfrarotstrahlung, die mit dem Körper in Wechselwirkung stand;
   e) Erzeugen eines den prozentualen Körperfettanteil wiedergebenden Ausgabesignals, das aus einem elektrischen Signal aus der Nahinfrarotabsorption des Detektors gebildet wird, gekennzeichnet durch Zurverfügungstellen eines Nahinfrarot-Mengenmeßgerätes (50), das weiterhin eine Auflageeinrichtung aufweist, mit der die wenigstens eine Punktlichtquelle (16") direkt gegen den zu überprüfenden Körper angelegt wird, so daß Nahinfrarotstrahlung in den Körper eingeleitet wird, wobei die Auflageeinrichtung den Detektor (28') zum Empfangen der Nah-infrarotstrahlung der Punktlichtquelle (16"), welche mit dem Körper in Wechselwirkung steht, und zur Bereitstellung des elektrischen Signals, das dem Körperfettanteil entspricht, positioniert.

2. Verfahren nach Anspruch 1, dadurch gekennzeichnet, daß die Nahinfrarotstrahlung innerhalb eines Bereiches von etwa 740 bis 1.100 Nanometer ist.

3. Verfahren nach Anspruch 1, dadurch gekennzeichnet, daß die Nahinfrarotabsorbtion bei etwa 950 Nanometer gemessen wird.

4. Verfahren nach Anspruch 1, dadurch gekennzeichnet, daß die Nahinfrarotabsorbtion bei zwei verschiedenen Wellenlängen gemessen wird.

5. Verfahren nach Anspruch 4, dadurch gekennzeichnet, daß eine der Wellenlängen um 937 Nanometer ± etwa 2 Nanometer liegt, und die andere der beiden Wellenlängen um 947 Nanometer ± etwa 2 Nanometer liegt, wobei ein Minimum von etwa 10 Nanometer zwischen den beiden Wellenlängen vorgesehen ist.

6. Verfahren nach Anspruch 1, dadurch gekennzeichnet, daß eine Vielzahl von physischen Parametern des Körpers neben der Absorbtion genutzt werden, um den Körperfettanteil quantitativ zu bestimmen.

7. Verfahren nach Anspruch 6, dadurch gekennzeichnet, daß die physischen Parameter aus einer Gruppe ausgewählt sind, bestehend aus Größe, Gewicht, Trainingsniveau, Geschlecht, Abstammung, Taille zur Hüftemaß, Armumfang, und Kombinatinen davon.

8. Verfahren nach Anspruch 6, dadurch gekennzeichnet, daß die physischen Parameter Größe und Gewicht sind.

9. Nahinfrarot-Mengenmeßgerät (50) zur Messung des Fettanteils eines zu überprüfenden Körpers, mit:

> a) wenigstens einer Punktlichtquelle (16") mit Nahinfrarotstrahlung;
> b) ein Nahinfrarotstrahlungsdetektor (28'), der ein elektrisches Signal bei Detektion einer nahinfraroten Strahlung ausgibt;
> c) eine Einrichtung (60), die verhindert, daß Nahinfrarotstrahlung von wenigstens einer Punktlichtquelle (16") direkt auf den Detektor (28') auftrifft; und
> d) eine Einrichtung (30',40',41',36') zum Wandeln eines elektrischen Signals, das dem Körperfettanteil entspricht, in einen Ausgabewert, der den prozentualen Körperfettanteil angibt, dadurch gekennzeichnet, daß das Meßgerät (50) weiterhin eine Auflageeinrichtung aufweist, mit der die wenigstens eine Punktlichtquelle (16") direkt an den zu überprüfenden Körper anlegbar ist, so daß Nahinfrarotstrahlung in den Körper einleitbar ist, wobei die Auflageeinrichtung den Detektor (28') zum Empfangen der Nahinfrarotstrahlung, von der wenigstens eine Punktlichtquelle (16"), welche mit dem Körper in Wechselwirkung steht, und zur Bereitstellung des elektrischen Signals, das dem Körperfettanteil entspricht, positioniert.

10. Meßgerät nach Anspruch 9, dadurch gekennzeichnet, daß wenigstens eine Punktlichtquelle mit nahinfraroter Strahlung eine IRED (16") aufweist.

11. Meßgerät nach Anspruch 9, dadurch gekennzeichnet, daß die wenigstens eine Punktlichtquelle eine IRED (16") und eine für Nahinfrarot transparente Fensterabdeckeinrichtung (56) aufweist, zum Anlegen an einen zu überprüfenden Körper, benachbart zur IRED (16") angeordnet.

12. Meßgerät nach Anspruch 9, dadurch gekennzeichnet, daß ein Gehäuse (55) vorgesehen ist, das die Auflageeinrichtung einen Abschnitt des Gehäuses umfaßt, in dem die wenigstens eine Punktlichtquelle (16") und der Detektor (28') angeordnet sind.

13. Meßgerät nach Anspruch 9, dadurch gekennzeichnet, daß ein Gehäuse (55) vorgesehen ist, daß die wenigstens eine Punktlichtquelle (16") mit nahinfraroter Strahlung in einem Ende des Gehäuses (55) angeordnet ist, wobei dieses Ende des Gehäuses (55) die Auflageeinrichtung zum Anlegen der wenigstens einen Punktlichtquelle an einen zu überprüfenden Körper aufweist; das der Nahinfrarot-Strahlungsdetektor (28') benachbart zur wenigstens einen Punktlichtquelle (16") in dem einen Ende des Gehäuses (55) angeordnet ist; und daß eine Einrichtung (30') mit dem Detektor (28') zum Verstärken des elektrischen Signals des Detektors (28') verbunden ist.

14. Meßgerät nach Anspruch 9, dadurch gekennzeichnet, daß die wenigstens eine Punktlichtquelle (16") mit nahinfraroter Strahlung innerhalb eines Bereiches von etwa 740 - 1.100 Nanometer emittiert.

15. Meßgerät nach Anspruch 9, dadurch gekennzeichnet, daß die wenigstens eine Punktlichtquelle (16") mit nahinfraroter Strahlung bei etwa 950 Nanometer emittiert.

16. Meßgerät nach Anspruch 9, dadurch gekennzeichnet, daß eine zweite Punktlichtquelle (16") mit nahinfraroter Strahlung vorgesehen ist.

17. Meßgerät nach Anspruch 16, dadurch gekennzeichnet, daß eine Punktlichtquelle (16") Strahlung um etwa 937 Nanometer ± etwa 2 Nanometer emittiert, und die andere Punktlichtquelle (16") Strahlung um etwa 947 Nanometer ± 2 Nanometer emittiert, wobei ein Minimum von etwa 10 Nanometer zwischen den beiden Wellenlängen liegt.

18. Meßgerät nach Anspruch 9, dadurch gekennzeichnet, daß die Einrichtung (30',40',41',36') zum Wandeln Datenwerte aus einer Vielzahl von physischen Parametern des Körpers verwendet, um den Körperfettanteil quantitativ festzustellen.

19. Meßgerät nach Anspruch 18, dadurch gekennzeichnet, daß die physischen Parameter aus einer Gruppe ausgewählt sind, bestehend aus Größe, Gewicht, Trainingszustand, Geschlecht, Abstammung, Taille zur Hüftemaß, Armumfang und Kombinationen davon.

20. Meßgerät nach Anspruch 18, dadurch gekennzeichnet, daß die physischen Parameter Größe und Gewicht sind.

**21.** Eine Kombination aus einem Nahinfrarot-Mengenmeßgerät zur Messung des Fettanteiles des zu überprüfenden Körpers und einer Reflektionsreferenz, mit:

a) einem Gehäuse (55);

b) einen Nahinfrarot-Strahlungsdetektor, der ein elektrisches Signal bei Detektion einer nahinfraroten Strahlung ausgibt;

c) eine Einrichtung (60), die verhindert, daß Nahinfrarot-Energie von jeder der Punktlichtquellen (16") direkt auf den Detektor (28') auftrifft;

d) eine Einrichtung (30'), die zur Verstärkung eines elektrischen Signals des Detektors (28') mit dem Detektor (28') verbunden ist; und

e) eine Einrichtung zur Meßdatenauswertung und Ableseanzeige (40',41',36'), die mit der Verstärkungseinrichtung (30') verbunden ist, und die das Verstärkungssignal auswertet, so daß ein Ablesewert bereitgestellt ist, der den prozentualen Körperfettanteil aufgrund der Detektion der nahen Infrarotstrahlung anzeigt, wobei die nahe Infrarotstrahlung innerhalb eines Bereiches von etwa 740 bis 1.100 Nanometer liegt, gekennzeichnet durch ein einzelnes Paar von nahe beinanderliegenden nahe Infrarotstrahlung erzeugenden Punktlichtquelleneinrichtungen (16"), die in einem Ende des Gehäuses (55) angeordnet sind zur Übertragung naher Infrarotstrahlung in den Körper, wobei das eine Ende des Gehäuses (55) eine Auflageeinrichtung zum Anlegen des Punktlichtquelleneinrichtungspaares (16") direkt an einen zu überprüfenden Körper aufweist, wobei ein Detektor (28') zwischen beiden Punktlichtquellen (16") in dem einen Ende des Gehäuses (55) angeordnet ist; und wobei eine Reflektionsreferenzhülseneinrichtung (70) über das eine Ende anlegbar ist, wobei die Hülseneinrichtung (70) eine reflektierende Oberfläche (72) eines bekannten vorbestimmten Reflektionsgrades aufweist.

**22.** Die Kombination nach Anspruch 21, dadurch gekennzeichnet, daß die reflektierende Oberfläche (72) eine Menge nahinfraroter Energie reflektiert, die im wesentlichen gleich einer Menge nahinfraroter Energie ist, die während der nahinfraroten Wechselwirkung mit einem Körper von etwa 24 % Körperfettanteil entspricht.

**23.** Ein Verfahren nach Anspruch 1, dadurch gekennzeichnet, daß die Detektion der Nahinfrarot-Strahlung eine Detektion der Nahinfrarot-Strahlung bei nur einer Wellenlänge umfaßt.

**Revendications**

**1.** Procédé pour déterminer le pourcentage de graisse dans un corps, comprenant les étapes suivantes :

(a) procurer un instrument de quantification d'un rayonnement proche des infrarouges (50) comprenant :

(i) au moins une source ponctuelle de rayonnement proche des infrarouges (16");

(ii) un détecteur de rayonnement proche des infrarouges (28') susceptible de Fournir un signal électrique lors de la détection d'un rayonnement proche des infrarouges;

(iii) des moyens (60) pour empêcher le rayonnement proche des infrarouges en provenance de ladite(lesdites) source(s) ponctuelle(s) (16") de tomber directement sur ledit détecteur (28'); et

(iv) des moyens pour convertir (30', 40', 41', 36') un signal électrique correspondant à la teneur en graisse du corps en une lecture indicative du pourcentage de graisse dans le corps;

(b) placer la(les) source(s) ponctuelle(s) (16") contre un corps à analyser avec le détecteur (28') positionné pour recevoir le rayonnement proche des infrarouges qui interagit avec ledit corps;

(c) transmettre le rayonnement proche des infrarouges dans ledit corps;

(d) détecter le rayonnement proche des infrarouges qui a interagit avec ledit corps;

(e) fournir une lecture du pourcentage de graisse du corps correspondant à un signal électrique indicatif de l'absorption du rayonnement proche des infrarouges procuré par ledit détecteur (28');

caractérisé en ce qu'on fournit un instrument de quantification du rayonnement proche des infrarouges (50) comprenant en outre des moyens de positionnement pour placer ladite(lesdites) source(s) ponctuelle(s) (16") directement contre un corps à analyser de façon à introduire le rayonnement proche des infrarouges dans ledit corps et en ce que lesdits moyens de positionnement positionnent ledit détecteur (28') pour recevoir le rayonnement proche des infrarouges en provenance de ladite(lesdites) source(s) ponctuelle(s) (16") qui interagit avec ledit corps et pour fournir le signal électrique correspondant au pourcentage de graisse dudit corps.

2. Procédé selon la revendication 1, dans lequel ledit rayonnement proche des infrarouges est dans la plage d'environ 740-1100 nanomètres.

3. Procédé selon la revendication 1, dans lequel l'absorption du rayonnement proche des infrarouges est mesurée à environ 950 nanomètres.

4. Procédé selon la revendication 1, dans lequel l'absorption du rayonnement proche des infrarouges est mesurée au niveau de deux longueurs d'onde différentes.

5. Procédé selon la revendication 4, dans lequel l'une desdites longueurs d'onde est environ 937 nanomètres ± environ 2 nanomètres, et l'autre desdites longueurs d'onde est environ 947 nanomètres ± environ 2 nanomètres, avec un minimum d'environ 10 nanomètres entre lesdites deux longueurs d'onde.

6. Procédé selon la revendication 1, dans lequel on utilise des données sur une multiplicité de paramètres physiques du corps avec ladite absorption pour déterminer quantitativement la teneur en graisse du corps.

7. Procédé selon la revendication 6, dans lequel lesdits paramètres physiques sont choisis dans le groupe comprenant la taille, le poids, le niveau d'exercice, le sexe, la race, la distance de la taille aux hanches, la circonférence des bras, et des combinaisons de ces paramètres.

8. Procédé selon la revendication 7, dans lequel lesdits paramètres physiques sont la taille et le poids.

9. Instrument de quantification d'un rayonnement proche des infrarouges (50) pour mesurer la teneur en graisse d'un corps à analyser, comprenant :

    (a) au moins une source ponctuelle (16") d'un rayonnement proche des infrarouges;

    (b) un détecteur de rayonnement proche des infrarouges (28') susceptible de fournir un signal électrique lors de la détection d'un rayonnement proche des infrarouges;

    (c) des moyens (60) pour empêcher le rayonnement proche des infrarouges en provenance de ladite(lesdites) source(s) ponctuelle(s) de tomber directement sur ledit détecteur (28'); et

    (d) des moyens pour convertir (30', 40', 41', 36') un signal électrique correspondant à la teneur en graisse du corps en une lecture indicative du pourcentage de graisse du corps;

caractérisé en ce que cet instrument (50) comprend en outre des moyens de positionnement pour placer ladite(lesdites) source(s) ponctuelle(s) (16") directement contre un corps à analyser de façon à introduire le rayonnement proche des infrarouges dans ledit corps et en ce que lesdits moyens de positionnement positionnent ledit détecteur (28') pour recevoir le rayonnement proche des infrarouges en provenance de ladite(lesdites) source(s) ponctuelle(s) (16") qui interagit avec ledit corps et pour fournir le signal électrique correspondant à la teneur en graisse dudit corps.

10. Instrument selon la revendication 9, dans lequel ladite(lesdites) source(s) ponctuelle(s) de rayonnement proche des infrarouges comporte(nt) une diode d'émission d'infrarouges (IRED) (16").

11. Instrument selon la revendication 9, dans lequel ladite(lesdites) source(s) ponctuelle(s) comporte(nt) une IRED (16") et un moyen de recouvrement sous forme d'une fenêtre transparente à un rayonnement proche des infrarouges (56) pour placer contre un corps à analyser, disposé adjacent à cette IRED (16").

12. Instrument selon la revendication 9, comprenant en outre un boîtier (55) lesdits moyens de positionnement comprenant une portion dudit boîtier à l'intérieur de laquelle sont disposés ladite(lesdites) source(s) ponctuelle(s) (16") et ledit détecteur (28').

13. Instrument selon la revendication 9, comprenant en outre :

un boîtier (55);

au moins une source ponctuelle (16") de rayonnement proche des infrarouges disposée dans une extrémité dudit boîtier (55), cette extrémité dudit boîtier (55) comprenant lesdits moyens de positionnement pour positionner cette source ponctuelle contre un corps à analyser;

ledit détecteur de rayonnement proche des infrarouges (28') étant positionné adjacent à cette source ponctuelle (16") dans cette extrémité dudit boîtier (55); et

des moyens (30') reliés au détecteur (28') pour amplifier ledit signal électrique fourni par ledit détecteur (28').

14. Instrument selon la revendication 9, dans lequel ladite(lesdites) source(s) ponctuelle(s) (16") de rayonnement proche des infrarouges émet(émettent) un rayonnement à l'intérieur de la plage d'environ 740-1100 nanomètres.

15. Instrument selon la revendication 9, dans lequel ladite(lesdites) source(s) ponctuelle(s) (16") de rayonnement proche des infrarouges émet(émettent) un rayonnement à environ 950 nanomètres.

16. Instrument selon la revendication 9, comprenant en outre une deuxième source ponctuelle (16") de rayonnement proche des infrarouges.

17. Instrument selon la revendication 16, dans lequel une source ponctuelle (16") émet un rayonnement à environ 937 nanomètres ± environ 2 nanomètres, et l'autre source ponctuelle (16") émet un rayonnement à environ 947 nanomètres ± environ 2 nanomètres, avec un minimum d'environ 10 nanomètres entre lesdites deux longueurs d'onde.

18. Instrument selon la revendication 9, dans lequel lesdits moyens de conversion (30', 40', 41', 36') utilisent des données sur une multiplicité de paramètres physiques du corps pour déterminer quantitativement la teneur en graisse du corps.

19. Instrument selon la revendication 18, dans lequel lesdits paramètres physiques sont choisis dans le groupe comprenant la taille, le poids, le niveau d'exercice, le sexe, la race, la distance de la ceinture aux hanches, la circonférence des bras et des combinaisons de ces paramètres.

20. Instrument selon la revendication 18, dans lequel lesdits paramètres physiques sont la taille et le poids.

21. En combinaison, un instrument de quantification d'un rayonnement proche des infrarouges pour mesurer la teneur en graisse d'un corps à analyser et un étalon réfléchissant, comprenant :

(a) un boîtier (55);

(b) un détecteur de rayonnement proche des infrarouges susceptible de fournir un signal électrique lors de la détection d'un rayonnement proche des infrarouges;

(c) des moyens pour empêcher (60) une énergie proche des infrarouges en provenance de chacun desdits moyens de sources ponctuelles (16") de tomber directement sur ledit détecteur (28');

(d) des moyens (30') reliés au détecteur (28') pour amplifier un signal électrique fourni par ledit détecteur (28'); et

(e) des moyens de traitement des données et de lecture (40', 41', 36') reliés aux moyens d'amplification (30') et susceptibles de traiter le signal amplifié de façon à procurer une lecture indicative du pourcentage de graisse dans le corps sur la base de la détection du rayonnement proche des infrarouges, dans lesquels ledit rayonnement proche des infrarouges est dans la plage d'environ 740-1100 nanomètres,

caractérisé en ce que
une seule paire de moyens de source ponctuelle produisant un rayonnement proche des infrarouges et étroitement accordés (16") est disposée dans une extrémité dudit boîtier (55) pour transmettre le rayonnement proche des infrarouges dans le corps, cette extrémité dudit boîtier (55) comprenant des moyens de positionnement pour positionner ladite paire de moyens de sources ponctuelles (16") directement contre le corps à analyser;
le détecteur (28') étant positionné entre chacun desdits moyens de source ponctuelle (16") dans cette extrémité dudit boitier (55); et

des moyens de manchon étalon réfléchissant (70) à positionner sur cette extrémité, lesdits moyens de manchon (70) comprenant une surface réfléchissante (72) d'une réflectance prédéterminée connue.

22. Combinaison selon la revendication 21, dans laquelle ladite surface réfléchissante (72) réfléchit l'énergie proche des infrarouges en une quantité sensiblement égale à la quantité d'énergie proche des infrarouges transmise pendant une interaction du rayonnement proche des infrarouges en provenance d'un corps ayant un pourcentage d'environ 24% de graisse.

23. Procédé selon la revendication 1, dans lequel l'étape consistant à détecter le rayonnement proche des infrarouges consiste à détecter un rayonnement proche des infrarouges au niveau de seulement une longueur d'onde.

1/5

FIG. 1

FIG. 2

FIG. 3

FIG.4A

EP 0 408 637 B1

FIG.4B

LOG I/I @ 947nm

PERCENT BODY FAT

N = 14
C = -0.91

FIG. 6

FIG. 5

FIG. 7

FIG. 8